# FASCICULE DE BREVET EUROPEEN

(11) **EP 1 461 121 B1**
(45) Date de publication et mention de la délivrance du brevet: **05.10.2011**
(21) Numéro de dépôt: 02793111.2
(22) Date de dépôt: 23.12.2002
(51) Int. Cl.: A61N 1/39

(54) **Dispositif d'ajustage de l'énergie de défibrillation par rapport à la résistance transthoracique d'un patient**
Vorrichtung zur Einstellung der Defibrillierungsenergie bezüglich des transthorakalen Widerstandes eines Patienten
Device for adjusting defibrillation energy relative to a patient's transthoracic resistance

(30) Priorité: 28.12.2001 FR 0117019
(43) Date de publication de la demande: 29.09.2004
(73) Titulaire: Schiller Medical SA, 67160 Wissembourg (FR)
(72) Inventeur: CANSELL, Albert, F-67170 Wissembourg (FR); DASKALOV, Ivan, 1408 Sofia (BG)
(74) Mandataire: Hepp Wenger Ryffel AG
(86) Numéro de dépôt international: PCT/EP2002/014723
(87) Numéro de publication internationale: WO 2003/055557

(56) Documents cités:
- EP-A- 0 437 104
- EP-A- 0 526 175
- EP-A- 1 118 353
- FR-A1- 2 808 694
- FR-A1- 2 808 694
- GB-A- 2 349 091
- US-A- 5 593 427
- US-A- 5 593 427
- US-A- 5 991 658
- US-A- 6 096 063
- US-A- 6 096 063

## Description

La présente invention se situe dans le domaine médical, et concerne plus particulièrement la défibrillation cardiaque et la cardioversion, en application externe ou transthoracique et a pour objet une méthode de compensation et d'ajustage automatiques de l'énergie de défibrillation de manière à délivrer au patient toute l'énergie sélectionnée quelle que soit la résistance du patient entre les électrodes qui lui sont appliquées.

Actuellement il existe des défibrillateurs à ondes biphasiques et à ondes biphasiques hachées c'est-à-dire découpées pour former une série d'impulsions élémentaires à haute fréquence (voir par exemple: A. Cansell, La Revue des SAMU, 2000,20.280-284).

Une méthode possible permettant de prendre en considération la résistance du patient consiste à mesurer cette résistance avant l'application du choc de défibrillation par un courant de faible intensité et de haute fréquence et de régler automatiquement l'énergie pour un courant présélectionné (voir par exemple: Kerber et al. Circulation 1984, 70, 303-308; Dalzell et al. Brit Heart J 1989, 61, 502-505). Cette méthode a l'inconvénient de présenter une mesure de la résistance selon une précision limitée (de l'ordre de ±10-15 Ohm) et d'exiger un temps de l'ordre de quelques secondes qui vient s'ajouter à la durée de charge du condensateur pour délivrer le choc.

Une autre méthode consiste à mesurer le début de la pente descendante d'une impulsion de défibrillation non-découpée, pente à partir de laquelle la résistance du patient est calculée en vue d'allonger la durée de cette impulsion jusqu'à ce que l'énergie présélectionnée ait été délivrée (voir US Patent No. 5593427). Chez des patients à haute résistance, l'énergie continue ainsi d'être appliquée à des durées d'impulsion de plus de 5 à 6 ms et même jusqu'à 10 ms pour la première phase, ce qui n'est physiologiquement pas adéquat. L'énergie délivrée au-delà d'une durée d'environ 5 ms est inefficace et éventuellement nocive et peut, dans certains cas, engendrer une refibrillation. En outre, pour des impulsions biphasiques, la répartition des énergies en phase 1 et phase 2 est différente pour différentes résistances de patients. Il en résulte que le rapport entre les deux phases en sera modifié et ne pourra de ce fait pas avoir sa valeur optimale (cf. par exemple: A. Cansell, La Revue des SAMU, 2000,20.280-284).

Une modification de cette méthode évite ce dernier inconvénient et permet un rapport optimal entre les amplitudes des deux phases tout en limitant leurs durées à un maximum de 4 à 5 ms par phase et en appliquant la mesure et la compensation à chacune des deux phases (voir Krasteva et al. J. Med. Eng. Technol. 24, 210-214). De cette manière le potentiel transmembranaire s'élève au dessus du seuil de défibrillation en moins de 4 à 5 ms et retourne à son niveau initial vers la fin de l'impulsion de phase 2. L'insuffisance de cette solution est la difficulté de maintenir une compensation adéquate avec des impulsions aussi courtes (4 à 5 ms) pour des résistances de patients situées dans la gamme de 100 a 150 Ohm, ayant en vue que des valeurs jusqu'à environ 180 Ohm sont possibles.

Une troisième méthode consiste en l'application d'une impulsion de tension réduite suivie de la mesure du courant correspondant, obtenant ainsi la résistance, à partir de laquelle un courant présélectionné est forcé à travers le thorax, du patient (voir: Mittal et al. J. Am. college of Cardiology, 1999, 34, 1595-1601). La mesure du courant avec une tension en dessous du seuil de défibrillation peut donner des valeurs de résistance de patient différentes, en général plutôt supérieures à celles obtenues avec la tension réellement appliquée. En outre, cette solution ne tient pas compte du rapport optimal des amplitudes ou des énergies des deux phases, qui n'est pas obtenu de façon adéquate. La deuxième phase ne fait que décharger sur le patient l'énergie restante de la phase 1, de sorte que l'amplitude de la deuxième phase est imposée par ce reste d'énergie. La présente invention a pour but de pallier certains des inconvénients et limitations précités.

Les inventeurs ont déjà crée et prouvé l'efficacité d'une catégorie d'impulsions biphasiques découpées ou hachées en impulsions élémentaires modulées en fréquence ou en durée, ou les deux, qui permettent d'obtenir des ondes moyennées d'énergie ou de courant de différentes formes et allures tel que décrit dans la demande de brevet publiée en France sous le n° 2808694.

Les inventeurs ont trouvé qu'après avoir mesuré la résistance du patient en utilisant la première impulsion élémentaire réelle appliquée au patient, les impulsions élémentaires subséquentes pouvaient être modulées de manière à délivrer au patient l'énergie qui a été présélectionnée, et ceci indépendamment de la résistance transthoracique, sans avoir à utiliser des durées totales de chaque phase de plus de 4 à 5 ms. Cette durée étant optimale pour un maximum d'efficacité, une telle compensation s'avère importante. En effet, comme on applique au patient une tension, si la résistance du patient augmente, le courant traversant le patient va baisser proportionnellement et l'énergie appliquée au patient va baisser fortement. Le principe proposé par la présente invention a l'avantage d'aboutir à une énergie délivrée sensiblement constante correspondant à la valeur de référence donnée sur 50 Ohm et voulue par l'opérateur, sans allonger la durée totale de l'impulsion de défibrillation qui est l'enveloppe du train d'impulsions comme il est le plus souvent pratiqué. L'invention concerne donc un dispositif pour générer un tel signal.

Une manière pratique de prévoir la compensation de l'énergie appliquée pour toute la gamme de résistances des patients s'étendant entre par exemple 40 et 180 Ω consiste à segmenter cette gamme en un certain nombre de plages de résistances et d'associer à chaque plage de résistances une modulation selon une loi préétablie (aussi appelée ci-après une loi de découpage, de hachage ou de modulation), prévue pour compenser et ajuster la valeur de l'énergie en fonction de la résistance d'un patient donné.

L'invention est définie dans la revendication indépendante 1. Des modes de réalisation préférés sont définis dans les revendications dépendantes.

L'invention sera mieux comprise grâce à la description ci-après, qui se rapporte à des modes de réalisation préférés, donnés à titre d'exemples non limitatifs et expliqués en référence aux dessins schématiques annexés, dans lesquels :
la figure 1 est un schéma-bloc fonctionnel général illustrant le principe de base de l'invention,
la figure 2 est le schéma détaillé du circuit de haute tension du dispositif générateur selon l'invention,
les figures 3, 4, 5 et 6 sont des graphiques représentant quelques exemples d'impulsions de défibrillation découpées ou hachées et modulées correspondant chacun à des résistances différentes de patients, et
la figure 7 est un graphique comparatif montrant la perte relative d'énergie en pourcentage en fonction de la résistance du patient, avec et sans la compensation résultant de l'invention.

La présente description décrit l'idée générale selon laquelle un procédé de compensation et d'ajustage automatiques de l'énergie de défibrillation ou de cardioversion en vue de la délivrance par l'intermédiaire d'électrodes de défibrillation reliées à un défibrillateur d'au moins une impulsion de défibrillation de la valeur totale de l'énergie sélectionnée sous la forme d'au moins une impulsion de défibrillation ou phase de polarité donnée constituée d'au moins une série d'impulsions élémentaires est caractérisé en ce que l'on mesure la résistance transthoracique du patient entre les deux électrodes appliquées au patient en utilisant la première impulsion élémentaire de l'impulsion de défibrillation, et en ce que les impulsions élémentaires suivantes et les pauses consécutives sont modulées selon une loi de modulation de manière à délivrer au patient l'énergie qui a été présélectionnée ou prévue et ceci quelle que soit sa résistance transthoracique mesurée entre les électrodes de défibrillation et en gardant sensiblement constante la durée totale de l'enveloppe de la série d'impulsions élémentaires de défibrillation correspondant à au moins une phase de polarité donnée.

Ce procédé se caractérise aussi en ce que l'étendue des résistances connues de patients est partagée en plusieurs gammes et en ce qu'à chaque gamme correspond une loi de modulation distincte.

Ce procédé se caractérise aussi en ce qu'une phase de polarité donnée a une durée prédéterminée.

Ce procédé se caractérise aussi en ce que la durée déterminée de chaque phase de polarité donnée est de 4 à 5 ms.

Ce procédé se caractérise encore en ce que l'impulsion de défibrillation est une impulsion de défibrillation biphasique à deux phases de polarité donnée différente découpée en impulsions élémentaires dont la première est utilisée pour la mesure de la résistance transthoracique du patient en vue de moduler les suivantes pour la délivrance d'une énergie dedéfibrillation prédéterminée.

Dans le cas d'un fonctionnement en onde biphasique, le dimensionnement libre de l'amplitude de la deuxième phase au moyen d'un deuxième condensateur que l'on charge à la tension voulue est un moyen d'obtenir une proportion donnée entre l'amplitude notamment en courant, en charge électrique (produit de l'intensité par le temps) ou en énergie de la deuxième phase par rapport à la première phase si les durées des deux phases doivent rester constantes. Par contre, si l'on utilise un seul condensateur, l'amplitude de la deuxième phase sera égale à l'amplitude de la première phase, que l'on inverse, et les proportions du courant, de la charge électrique (produit de l'intensité par le temps) ou de l'énergie des deux phases ne pourront pas être choisies librement.

Il existe cependant une manière d'éviter l'utilisation de deux condensateurs tout en gardant la possibilité d'obtenir une proportion prédéterminée de la deuxième phase par rapport à la première phase, proportion exprimée soit en unités de charge électrique, soit en énergies des deux phases.

Cette manière consiste en utilisant un seul condensateur, à doser de façon adéquate la charge totale ou l'énergie totale nécessaire à la deuxième phase en agissant sur les durées soit des impulsions individuelles de cette deuxième phase soit sur celles des pauses de cette deuxième phase, soit sur les deux à la fois, sans modifier la durée totale de cette deuxième phase. On peut ainsi décharger une énergie prédéterminée dans un temps donné.

La loi de découpage et de modulation choisie peut consister par exemple - pour une loi donnée - en une fréquence fixe et un rapport cyclique (rapport entre la durée de l'impulsion et la durée du cycle) constant (pour une loi de découpage et de modulation donnée). Selon le contenu d'énergie à obtenir dans les deux phases, ce rapport cyclique constant - pour une loi de modulation donnée - peut prendre toutes les valeurs entre 0%, et 100 %.

Si pour des raisons techniques ou autres le rapport cyclique constant choisi pour une loi de modulation donnée a intérêt à être le même pour les deux phases, il ne sera normalement pas possible d'agir librement et indépendamment sur le contenu de charge électrique (ou d'énergie) contenu dans la deuxième phase afin que celui-ci ait une proportion voulue par rapport au contenu de charge électrique (ou d'énergie) de la première phase, si en plus on s'efforce de respecter la conditions de départ d'avoir une durée de la deuxième phase constante et égale à la première, de 4 à 5 ms.

Dans le but de chercher une solution à ce problème, des expérimentations, et études récentes plus approfondies ont été menées par les inventeurs en ce qui concerne les durées physiologiquement optimales et la constitution (découpée ou non) des deux phases. Ces études ont apporté les enseignements suivants.Ils ont confirmé d'une part qu'en ce qui concerne la première phase, pour avoir une efficacité la plus élevée possible, elle devrait être découpée selon les principes développés plus haut et avoir comme indiqué une durée de 4 à 5 ms. La raison de cet enseignement réside dans le fait que physiologiquement c'est la première phase qui est essentiellement responsable de l'effet de défibrillation. C'est la part active de la défibrillation, pour laquelle le découpage joue un rôle très important ainsi que la durée de la phase, qui est conditionnée par le temps d'excitation des cellules. D'autre part,en ce qui concerne la deuxième phase par contre, elle n'intervient pas directement dans l'action de défibrillation, mais n'a pour but que d'éliminer les excès de charges électriques accumulées dans le coeur par suite de la première phase. L'élimination de ces charges a pour conséquence d'éviter des phénomènes de refibrillation, ce qui permet de maintenir l'effet de défibrillation obtenu par la 1^{ére} phase. Ce fait déjà connu pour des ondes biphasiques traditionnelles non découpées a été confirmé par les inventeurs si l'on utilise une première phase découpée. Les inventeurs ont alors émis l'hypothèse que par suite de ce rôle plutôt passif de la 2^{éme} phase, le fait du découpage et celui de la durée de cette phase ne jouent peut-être pas de rôle. Cette hypothèse s'est vue confirmée par les études des inventeurs : ils ont constaté qu'après une première phase découpée et bien dimensionnée pour défibriller, la deuxième phase pouvait indifféremment être, soit découpée et avoir une durée dont la valeur peut être variable sans que cela ait une influence sur l'efficacité, à condition que son contenu de charge (ou d'énergie) ait une proportion, adéquate par rapport au contenu de charge (ou d'énergie) de la 1^{ère} phase, soit non découpée en ayant une durée dont la valeur peut également être variable sans que cela ait une influence sur l'efficacité, à condition que son contenu de charge (ou d'énergie) ait une proportion adéquate par rapport au contenu de charge (ou d'énergie) de la 1^{ère} phase.

En ce qui concerne la mesure de résistance au moyen de la première impulsion et au choix consécutif d'une loi de découpage et de modulation, les inventeurs ont trouvé qu'il pouvait être avantageux que la mesure de la résistance du patient et le choix de la loi de découpage et de modulation pouvaient se faire au cours même de la première impulsion. De cette manière le premier cycle (impulsion - pause) pourra déjà utiliser la loi de modulation choisie et en faire partie intégrante, de telle sorte que ce premier cycle puisse déjà avoir par exemple un rapport cyclique identique à un autre de la série, ou être identique à tous les cycles si le rapport cyclique a été choisi constant comme dans l'exemple donné plus haut.

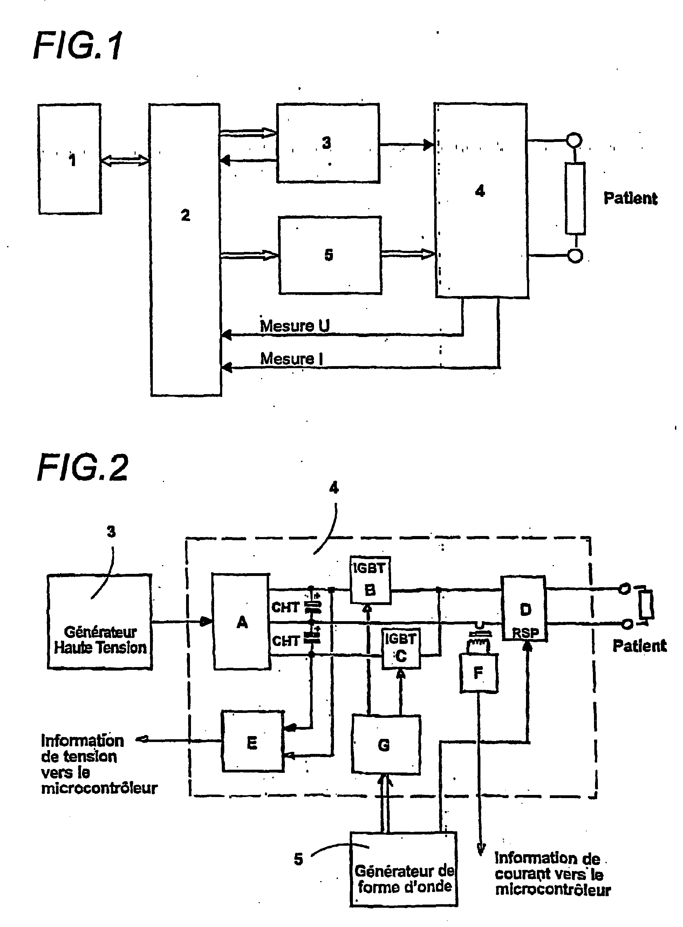

En référence à la figure 1 le schéma de base illustrant le principe de base de l'invention se compose des éléments suivants.

Sont montés selon le schéma de la figure 1 une interface utilisateur 1, un microcontrôleur 2, un générateur de haute tension 3, un circuit de haute tension 4 et un générateur de forme d'onde 5 dont la description est donnée ci-après. Le circuit de haute tension 4 est relié au microcontrôleur 2 par une liaison de mesure de la tension et par une liaison de mesure de l'intensité référencées respectivement mesure U et mesure I.

L'interface utilisateur 1 est un sous-ensemble qui réalise l'interfaçage entre les organes extérieurs de commande et l'unité de défibrillation.

Le microcontrôleur 2 est une unité fonctionnelle qui effectue l'ensemble des opérations nécessaires pour délivrer un choc de défibrillation lorsque celui-ci est indiqué. Il génère l'ensemble des signaux de commande nécessaires à la charge et à la décharge du ou des condensateur(s) de haute tension CHT par l'intermédiaire des sous-ensembles générateurs de haute tension 3, circuit de haute tension 4, et générateur de forme d'onde 5.

Le microcontrôleur 2 pilote le générateur de haute tension 3 afin de charger un ou plusieurs condensateur(s) de haute tension CHT jusqu'à la valeur d'énergie présélectionnée. Lorsque l'énergie présélectionnée est atteinte, le microcontrôleur 2 valide la décharge du ou des condensateur(s) de haute tension CHT. Lors du choc, le microcontrôleur 2 pilote le relais de sécurité patient référencé RSP ainsi que le générateur de forme d'onde 5 qui commande les commutateurs haute tension IGBT générant l'onde de défibrillation. Pendant le choc, le microcontrôleur 2 détermine la valeur de la résistance présentée par le patient à partir de la mesure des informations de courant et de tension donnée par la première impulsion élémentaire afin de réaliser la compensation et l'ajustage en énergie de l'onde de défibrillation par exemple biphasique en fonction de la résistance du patient.

Le générateur de haute tension 3 est piloté par le microcontrôleur 2. Il réalise le transfert de l'énergie de la source d'alimentation vers le ou (les) condensateurs haute tension CHT jusqu'à atteindre la valeur de l'énergie présélectionnée.

Le circuit de haute tension 4 réalise la décharge du ou (des) condensateurs CHT via les circuits de commutation à commutateurs IGBT et le relais patient référencé RSP. Le circuit de haute tension 4 est piloté par les deux sous-ensembles suivants : le microcontrôleur 2 ainsi que le générateur de forme d'onde 5. Afin d'effectuer la décharge de sécurité du ou des condensateur(s) CHT dans tous les cas, le circuit de haute tension 4 comprend également un circuit de décharge de sécurité. Le circuit de haute tension 4 réalise par ailleurs la mesure de la tension de charge du ou des condensateur(s) CHT ainsi que la mesure du courant traversant le patient lors du choc de défibrillation.

Le générateur de forme d'onde 5 réalise la commande des circuits de commutation à commutateurs IGBT afin de générer l'onde biphasique pulsée. Le générateur de forme d'onde 5 est piloté par le microcontrôleur 2 pour réaliser la compensation de l'onde biphasique pulsée en fonction de la résistance du patient.

On décrira ci-après à titre d'exemple non limitatif, le détail du circuit de haute tension 4 par ses blocs fonctionnels représentés sur le schéma de la figure 2.
Il se compose des blocs référencés A, B, C, D, E et F sur la figure 2.
A. Transformateur et redresseur haute tension
B. Interrupteur à semi-conducteur
C. Interrupteur à semi-conducteur
D. Interrupteur électromécanique
E. Mise en forme de l'information de tension.
F. Mise en forme de l'information de courant
G. Mise en forme des informations de commande des IGBT.
On indiquera ci-après le rôle de chacun de ces modules fonctionnels.

Le transformateur et redresseur haute tension A effectue le transfert d'énergie entre la source d'alimentation et les deux condensateurs haute tension CHT pouvant se réduire à un seul condensateur, par l'intermédiaire du générateur de haute tension 3. Par sa configuration, le sous-ensemble transformateur et redresseur haute tension permet la charge simultanée du ou des deux condensateurs haute tension CHT à la valeur de l'énergie préalablement sélectionnée.

Les deux sous-ensembles interrupteurs à semi-conducteurs B et C réalisent la génération de l'onde de défibrillation biphasique à impulsions élémentaires servant au choc par l'intermédiaire des signaux de pilotage issus du sous-ensemble générateur de forme d'onde 5. Ces deux sous-ensembles utilisent par exemple des commutateurs semi-conducteurs connus sous le sigle IGBT pour réaliser cette fonction. Chaque sous-ensemble B ou C peut être constitué d'une association de plusieurs commutateurs IGBT.

Le sous-ensemble D interrupteur électromécanique permet de réaliser l'isolation galvanique du patient en réduisant les courants de fuite internes aux interrupteurs à semi-conducteurs IGBT utilisés ici. Ce sous-ensemble D interrupteur électromécanique est un relais piloté par le générateur de formes d'onde 5 lors de l'application du choc de défibrillation. Il est appelé dans cette description relais de sécurité patient RSP.

Le sous-ensemble E de mise en forme de l'information de tension réalise la mesure de la tension de charge indiquée comme mesure U sur la figure 1 correspondant au seul condensateur ou aux deux condensateurs haute tension CHT afin que celle-ci puisse être exploitée par le microcontrôleur 2 pour la détermination de la résistance du patient.

Le sous-ensemble F de mise en forme de l'information de courant réalise la mesure isolée du courant de défibrillation lors du début du choc de défibrillation indiquée par mesure de I sur la figure 1. L'exploitation des deux informations de tension et de courant permet au microcontrôleur 2 de déterminer la résistance du patient lors du choc de défibrillation afin d'adapter le facteur de forme et plus généralement la modulation d'impulsion de l'onde de défibrillation par exemple biphasique.

Le sous-ensemble G de mise en forme des informations de commande réalise l'interfaçage et l'isolation galvanique nécessaires aux signaux de pilotage générés par le générateur de forme d'onde 5 afin de commander les interrupteurs à semi-conducteur B et C. Les signaux issus de ce sous-ensemble G provoquent la conduction ainsi que le blocage actif des deux sous-ensembles à semi-conducteurs IGBT afin de générer la forme d'onde de défibrillation adaptée à la résistance du patient lors de la défibrillation.

Bien entendu, l'association des blocs fonctionnels décrite ci-dessus ne constitue qu'un exemple parmi d'autres pouvant assurer la même fonction générale.

Ainsi, dans le cas d'un seul condensateur, une structure en pont permettant de le décharger sous deux phases de polarité différentes ou tout autre montage ou disposition de circuit entre pleinement dans le cadre de la présente invention.

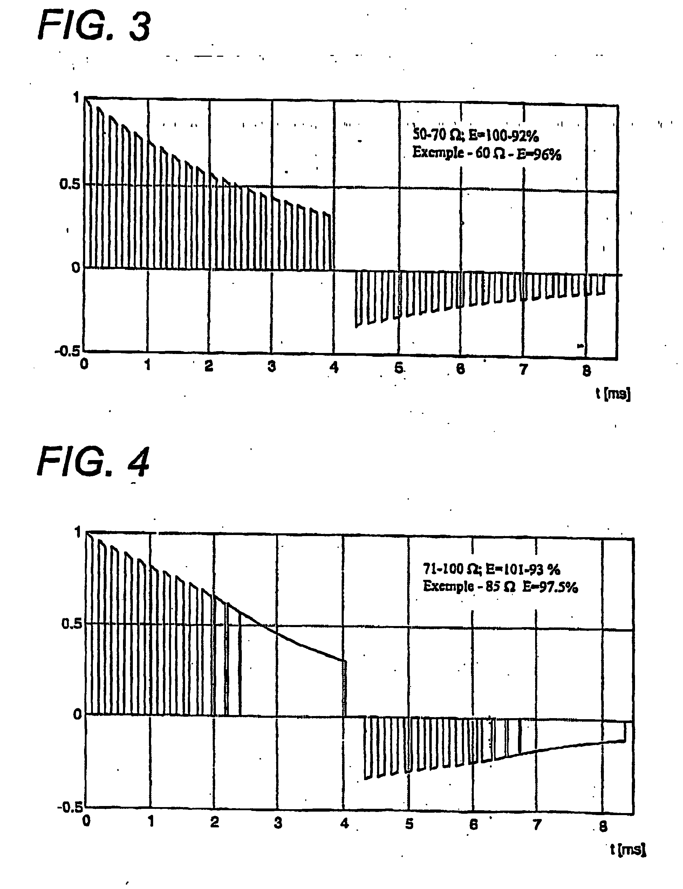

Les courbes des exemples représentés sur les figures 3 à 6 représentent quelques exemples d'impulsions de défibrillation découpées ou hachées, selon des impulsions élémentaires correspondant chacun à des résistances différentes, regroupées en plusieurs sous-ensembles par une succession de gammes de valeurs. La première impulsion élémentaire sert à mesurer la résistance transthoracique comme expliqué ci-dessus. Les impulsions élémentaires subséquentes sont modulées de manière à assurer l'application de l'énergie présélectionnée pour différentes résistances de patients.

La figure 3 donne la forme de l'impulsion de défibrillation correspondant à la plage de 40 à 70 Ohm. La courbe représentée correspond plus particulièrement à une résistance de patient de 60 Ohm. On remarque que le facteur de forme des impulsions de défibrillation dans ce domaine de valeurs de résistances proches de la valeur de référence de 50 Ohm est constant et égal à 1, c'est-à-dire qu'il n'est pas modulé. Le contenu total en énergie des trains d'impulsions élémentaires des deux phases est donc la valeur de référence sélectionnée ou prévue.

La figure 4 donne la forme de l'impulsion de défibrillation correspondant à la plage s'étendant de 71 à 100 Ohm. La courbe représentée correspond plus particulièrement à une résistance de patient de 85 Ohm. On remarque que le facteur de forme des impulsions dans ce domaine de résistances est à présent variable, c'est-à-dire qu'il est modulé. On voit notamment que la dernière impulsion élémentaire de la première phase par exemple est très large. La précédente est moins large, et la pause entre les deux est très réduite. Et ainsi de suite jusqu'à la première impulsion qui a une largeur identique à la première impulsion élémentaire de la figure 3.

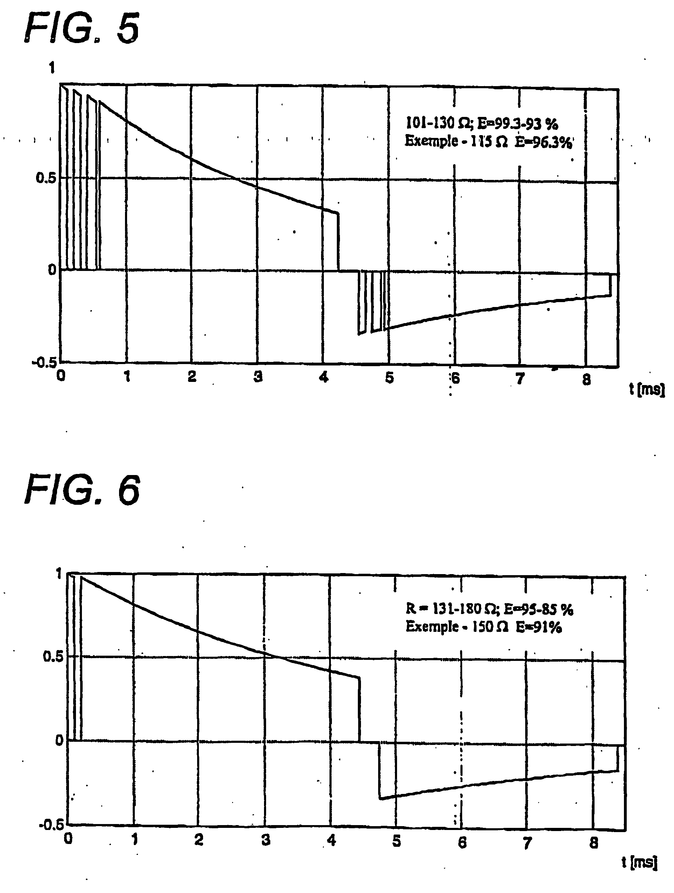

Les figures 5 et 6 correspondant respectivement à des plages de résistances de 101 à 130 Ohm et de 131 à 180 Ohm montrent un effet de remplissage de plus en plus important de l'enveloppe de la phase.

On remarque sur les figures 3 à 6 que la durée de chaque phase est sensiblement constante et comprise dans un intervalle s'étendant jusqu'à environ 4 à 5 ms. Au fur et à mesure de l'augmentation de la résistance, les enveloppes des phases se remplissent à partir de la fin des phases. On comprend donc que le contenu d'énergie augmente ainsi, ce qui compense la perte due à la réduction du courant consécutive à l'augmentation de résistance.

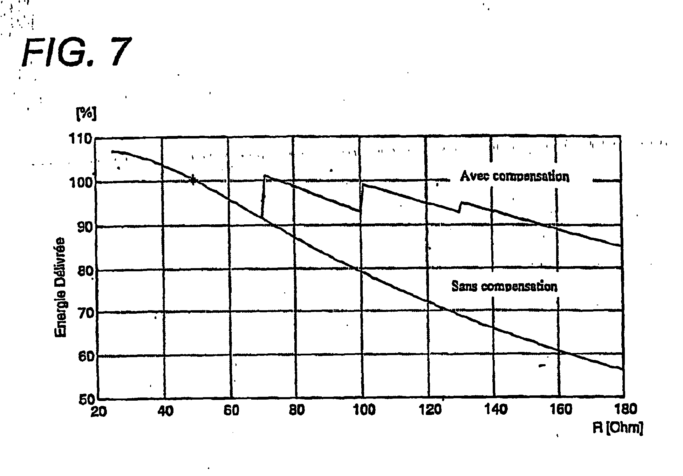

La figure 7 montre la perte d'énergie en pourcentage en fonction de la résistance du patient. Le point (*) indique l'emplacement de la courbe correspondant à 100% de l'énergie pour 50 Ohm qui est la référence habituelle prévue par les normes pour indiquer l'énergie.

La courbe en trait continu est la perte d'énergie sans compensation. La courbe en trait brisé montre la compensation d'énergie par changement de la modulation. Nous avons prévu dans l'exemple donné les quatre plages suivantes: de 40 à 70 Ω : sans compensation ; de 70 à 100 Ω - de 100 Ω à 130 Ω-de 130 à 180 Ω : avec compensation. La gamme de résistances peut bien entendu, comporter un plus grand nombre de plages, plus étroites, par exemple de 10 Ohm : 50 à 60 Ohm, 60 à 70 Ohm, etc.

L'invention ne se limite pas à une forme d'impulsion de défibrillation monophasique, mais couvre toutes celles connues et utilisées actuellement notamment les impulsions de défibrillation biphasiques.

Ce principe de compensation et d'ajustage de l'énergie en fonction de la résistance du patient convient pour toutes les applications de défibrillation. Que ce soit la défibrillation ventriculaire (en soins intensifs, en laboratoire d'électrophysiologie, ou en réanimation préhospitalière), ou en défibrillation implantable ou en cardioversion (défibrillation atriale).

## Revendications

1. Dispositif pour générer un signal de défibrillation, comportant des électrodes de défibrillation qui peuvent être appliquées à un patient ainsi que des moyens pour délivrer au moins un choc de défibrillation comportant au moins une série d'impulsions élémentaires, le dispositif comportant, par ailleurs,
• des moyens de mesure de la résistance du patient entre les électrodes appliquées au patient en utilisant la première impulsion élémentaire du signal de défibrillation
• des moyens pour stocker une pluralité de lois de découpage et de modulation destinées à moduler les largeurs des impulsions élémentaires et/ou des pauses entre ces impulsions : et/ou la fréquence de ces impulsions
• et des moyens pour choisir une loi de découpage en fonction de la résistance mesurée telle que l'énergie appliquée au patient soit compensée et ajustée à une valeur prédéterminée.

2. Dispositif selon la revendication 1, comportant un dispositif de compensation et d'ajustage automatique de l'énergie de défibrillation ou de cardioversion, **caractérisé en ce que** ledit dispositif comporte un circuit de haute tension (4) et **en ce que** le circuit de haute tension (4) se compose des sous-ensembles suivants: un transformateur et redresseur de haute tension (A), des moyens pour stocker l'énergie à délivrer au patient (CHT), deux interrupteurs à semi-conducteurs (BC) un interrupteur électromécanique (D), un module (E) de mise en forme de l'information de tension, un module (F) de mise en forme de l'information de courant et un module (G) de mise en forme des informations de commande.

3. Dispositif selon une des revendications 1 ou 2 **caractérisé en ce qu'**il comporte deux condensateurs.

4. Dispositif selon la revendication 1 **caractérisé en ce qu'**il comporte un seul condensateur.

5. Dispositif selon la revendication 4 **caractérisé en ce qu'**il comporte un montage en pont pour la décharge du seul condensateur.

6. Dispositif selon l'une quelconque des revendications précédentes, **caractérisé en ce que** les lois de découpage et de modulation stockées dans les moyens pour stocker sont telles que les impulsions élémentaires suivant la première impulsion élémentaire et les pauses consécutives sont modulées selon une loi de modulation de manière à former un signal dosé en énergie selon une quantité présélectionnée ou prévue d'énergie et ceci quelle que soit la résistance transthoracique mesurée du patient et en gardant sensiblement constante la durée totale de l'enveloppe de la série d'impulsions élémentaires de défibrillation correspondant à au moins une phase de polarité donnée.

7. Dispositif selon l'une quelconque des revendications précédentes, caractérisé que les moyens pour délivrer le choc de défibrillation sont adaptés pour délivrer une impulsion de défibrillation biphasique comportant deux phases différentes de polarité donnée différente.

8. Dispositif selon l'une quelconque des revendications précédentes, **caractérisé en ce que** les lois de découpage et de modulation stockées dans les moyens pour stocker sont telles que l'étendue des résistances connues de patients est partagée en plusieurs gammes et **en ce qu'**à chaque gamme correspond une loi de modulation distincte.

9. Dispositif selon la revendication 7 , **caractérisé en ce que** les moyens pour délivrer le choc de défibrillation sont adaptés pour délivrer une impulsion qui a, pour une phase de polarité donnée, une durée prédéterminée.

10. Dispositif selon la revendication 9 **caractérisé en ce que** la durée déterminée de chaque phase de polarité donnée est de 4 à 5 ms.

11. Dispositif selon les revendications 7 quand dépendante de la revendication 4 **caractérisé en ce que** les lois de découpage et de modulation stockées dans les moyens pour stocker sont telles qu'elles permettent d'agir sur les durées des impulsions individuelles de la deuxième phase de manière à doser la charge électrique totale ou l'énergie totale de cette deuxième phase pour que la charge électrique ou l'énergie de la deuxième phase ait une proportion voulue par rapport à la charge électrique ou à l'énergie de la première phase sans modifier la durée de cette deuxième phase.

12. Dispositif selon les revendications 7 quand dépendante de la revendication 4 **caractérisé en ce que**, les lois de découpage et de modulation stockées dans les moyens pour stocker sont telles d'agir sur les durées des pauses individuelles de la deuxième phase de manière à doser la charge électrique totale ou l'énergie totale de cette deuxième phase pour que la charge électrique ou l'énergie de la deuxième phase ait une proportion voulue par rapport à la charge électrique ou à l'énergie de la première phase sans modifier la durée de cette deuxième phase.

13. dispositif selon l'une quelconque des revendications 7, 9, 11 ou 12 **caractérisé en ce que** les lois de découpage et de modulation stockées dans les moyens pour stocker sont telles qu'elles permettent de choisir la durée de cette deuxième phase de manière à ce que son contenu de charge ou d'énergie ait une proportion prédéterminée par rapport au contenu de charge ou d'énergie de la première phase.

14. Dispositif selon l'une quelconque des revendications précédentes, **caractérisé en ce que** les moyens pour choisir une loi de découpage sont adaptés pour choisir cette loi au cours de la première impulsion, et sont de telle sorte que le cycle de cette première impulsion ait un rapport cyclique déterminé par rapport au moins un autre rapport cyclique de la série d'impulsions.

15. Dispositif selon l'une quelconque des revendications ou 9 **caractérisé en ce que** les lois de découpage et de modulation stockées dans les moyens pour stocker sont telles que la deuxième phase n'est pas découpée en impulsions élémentaires.

16. Dispositif selon la revendication 15 **caractérisé en ce que** les lois de découpage et de modulation' stockées dans les moyens pour stocker sont telles que la deuxième phase non découpée ait une durée telle que son contenu de charge ou d'énergie ait une proportion prédéterminée par rapport au contenu de charge ou d'énergie de la première phase découpée.

17. Dispositif selon l'une des revendications 14 à 16 **caractérisé en ce que** les lois de découpage et de modulation stockées dans les moyens pour stocker sont telles qu'elles permettent d'agir sur le rapport cyclique du cycle contenant la première impulsion pour qu'il ait une valeur prédéterminée en fonction de la résistance mesurée.

18. Dispositif selon la revendication 17, caractérisé en ce les lois de découpage et de modulation stockées dans les moyens pour stocker sont telles que le rapport cyclique du cycle contenant la première impulsion soit identique au rapport cyclique d'au moins un des cycles consécutifs

## Claims

1. Device for generating a defibrillation signal, including defibrillation electrodes that can be applied to a patient, as well as means for delivering at least one defibrillation shock including at least one series of elementary pulses, the device further including
• means for measuring the patient's resistance between the electrodes applied to the patient by using the first elementary pulse of the defibrillation signal
• means for storing a plurality of switching and modulation laws intended for modulating the widths of the elementary pulses and/or of the pauses between these pulses and/or the frequency of these pulses
• and means for selecting a switching law as a function of the measured resistance such that the energy applied to the patient is compensated and adjusted to a predetermined value.

2. Device according to Claim 1, including a device for automatically compensating and adjusting the defibrillation or cardioversion energy, **characterized in that** said device includes a high-voltage circuit (4) and **in that** the high-voltage circuit (4) is composed of the following subassemblies: a high-voltage transformer and rectifier (A), means for storing the energy to be delivered to the patient (HVC), two semiconductor switches (B, C), an electromechanical switch (D), a module (E) for conditioning the voltage information, a module (F) for conditioning the current information and a module (G) for conditioning the control information.

3. Device according to either of Claims 1 and 2, **characterized in that** it includes two capacitors.

4. Device according to Claim 1, **characterized in that** it includes a single capacitor.

5. Device according to Claim 4, **characterized in that** it includes a bridge layout for discharging the single capacitor.

6. Device according to any one of the preceding claims, **characterized in that** the switching and modulation laws stored in the storage means are such that the elementary pulses following the first elementary pulse and the consecutive pauses are modulated according to a modulation law so as to form a signal that is dosed in energy according to a preselected or intended amount of energy, and to do so irrespective of the patient's measured transthoracic resistance and while keeping substantially constant the total duration of the envelope of the series of elementary defibrillation pulses corresponding to at least one phase of given polarity.

7. Device according to any one of the preceding claims, **characterized in that** the means for delivering the defibrillation shock are adapted to deliver a biphasic defibrillation pulse including two different phases of different given polarity.

8. Device according to any one of the preceding claims, **characterized in that** the switching and modulation laws stored in the storage means are such that the spread of the known resistances of patients is divided into a plurality of ranges, and **in that** a distinct modulation law corresponds to each range.

9. Device according to Claim 7, **characterized in that** the means for delivering the defibrillation shock are adapted to deliver a pulse which, for a phase of given polarity, has a predetermined duration.

10. Device according to Claim 9, **characterized in that** the determined duration of each phase of given polarity is from 4 to 5 ms.

11. Device according to Claim 7 when dependent on Claim 4, **characterized in that** the switching and modulation laws stored in the storage means are such that they make it possible to control the durations of the individual pulses of the second phase so as to dose the total electric charge or the total energy of this second phase so that the electric charge or the energy of this second phase has a desired proportion with respect to the electric charge or the energy of the first phase, without modifying the duration of this second phase.

12. Device according to Claim 7 when dependent on Claim 4, **characterized in that** the switching and modulation laws stored in the storage means are such that they make it possible to control the durations of the individual pauses of the second phase so as to dose the total electric charge or the total energy of this second phase so that the electric charge or the energy of this second phase has a desired proportion with respect to the electric charge or the energy of the first phase, without modifying the duration of this second phase.

13. Device according to any one of Claims 7, 9, 11 and 12, **characterized in that** the switching and modulation laws stored in the storage means are such that they make it possible to select the duration of this second phase so that its charge or energy content has a predetermined proportion with respect to the charge or energy content of the first phase.

14. Device according to any one of the preceding claims, **characterized in that** the means for selecting a switching law are adapted to select this law during the first pulse, and are such that the cycle of this first pulse has a determined duty ratio with respect to at least one other duty ratio of the series of pulses.

15. Device according to either of Claims 7 and 9, **characterized in that** the switching and modulation laws stored in the storage means are such that the second phase is not switched into elementary pulses.

16. Device according to Claim 15, **characterized in that** the switching and modulation laws stored in the storage means are such that the unswitched second phase has a duration such that its charge or energy content has a predetermined proportion with respect to the charge or energy content of the switched first phase.

17. Device according to one of Claims 14 to 16, **characterized in that** the switching and modulation laws stored in the storage means are such that they make it possible to control the duty ratio of the cycle containing the first pulse so that it has a predetermined value dependent on the measured resistance.

18. Device according to Claim 17, **characterized in that** the switching and modulation laws stored in the storage means are such that the duty ratio of the cycle containing the first pulse is identical to the duty ratio of at least one of the consecutive cycles.

## Patentansprüche

1. Vorrichtung, um ein Defibrillationssignal zu erzeugen, die Defibrillationselektroden, die an einem Patienten angebracht werden können, sowie Mittel zum Liefern wenigstens eines Defibrillationsstoßes, der wenigstens eine Reihe elementarer Impulse enthält, umfasst, wobei die Vorrichtung außerdem umfasst:
- Mittel zum Messen des Widerstands des Patienten zwischen den an dem Patienten angebrachten Elektroden unter Verwendung des ersten elementaren Impulses des Defibrillationssignals,
- Mittel zum Speichern mehrerer Unterteilungs- und Modulationsgesetze, die dazu bestimmt sind, die Breiten der elementaren Impulse und/oder die Pausen zwischen diesen Impulsen und/oder die Frequenz dieser Impulse zu modulieren,
- und Mittel zum Wählen eines Unterteilungsgesetzes als Funktion des gemessenen Widerstands, derart, dass die in den Patienten eingebrachte Energie kompensiert und auf einen vorgegebenen Wert eingestellt wird.

2. Vorrichtung nach Anspruch 1, mit einer Vorrichtung zum automatischen Kompensieren und Einstellen der Defibrillations- oder Kardioversionsenergie, **dadurch gekennzeichnet, dass** die Vorrichtung eine Hochspannungsschaltung (4) umfasst und das die Hochspannungsschaltung (4) aus den folgenden Untereinheiten aufgebaut ist: einem Hochspannungstransformator und -gleichrichter (A), Mitteln (CHT) zum Speichern der an den Patienten auszugebenden Energie, zwei Halbleiterunterbrechern (B, C), einem elektromechanischen Unterbrecher (D), einem Modul (E) zum Formen der Spannungsinformationen, einem Modul (F) zum Formen der Strominformationen und einem Modul (G) zum Formen der Steuerinformationen.

3. Vorrichtung nach einem der Ansprüche 1 oder 2, **dadurch gekennzeichnet, dass** sie zwei Kondensatoren umfasst.

4. Vorrichtung nach Anspruch 1, **dadurch gekennzeichnet dass** sie einen einzigen Kondensator umfasst.

5. Vorrichtung nach Anspruch 4, **dadurch gekennzeichnet, dass** sie eine Brückenschaltung für die Entladung des einzigen Kondensators umfasst.

6. Vorrichtung nach einem der vorhergehenden Ansprüche, **dadurch gekennzeichnet, dass** die Unterteilungs- und Modulationsgesetze, die in den Speichermitteln gespeichert sind, derart sind, dass die dem ersten elementaren Impuls folgenden elementaren Impulse und die aufeinander folgenden Pausen gemäß einem Modulationsgesetz moduliert werden, derart, dass ein Signal gebildet wird, dessen Energie entsprechend einer im Voraus gewählten Größe dosiert ist oder das entsprechend mit Energie versehen ist, unabhängig von dem gemessenen ohmschen Widerstand durch den Brustkorb des Patienten und im Wesentlichen unter Konstanthaltung der Gesamtdauer der Einhüllenden der Reihe elementarer Defibrillationsimpulse, die wenigstens einer gegebenen Polarisationsphase entspricht.

7. Vorrichtung nach einem der vorhergehenden Ansprüche, **dadurch gekennzeichnet, dass** die Mittel zum Liefern des Defibrillationsstoßes dazu ausgelegt sind, einen zweiphasigen Defibrillationsimpuls auszugeben, der zwei verschiedene Phasen mit unterschiedlicher gegebener Polarität enthält.

8. Vorrichtung nach einem der vorhergehenden Ansprüche, **dadurch gekennzeichnet, dass** die Unterteilungs- und Modulationsgesetze, die in den Speichermitteln gespeichert sind, derart sind, dass der Bereich bekannter Widerstände von Patienten in mehrere Bereiche unterteilt wird und dass jeder Bereich einem unterschiedlichen Modulationsgesetz entspricht.

9. Vorrichtung nach Anspruch 7, **dadurch gekennzeichnet, dass** die Mittel zum Liefern des Defibrillationsstoßes dazu ausgelegt sind, einen Impuls zu liefern, der für eine Phase mit gegebener Polarität eine vorgegebene Dauer besitzt.

10. Vorrichtung nach Anspruch 9, **dadurch gekennzeichnet, dass** die bestimmte Dauer jeder Phase mit gegebener Polarität 4 bis 5 ms beträgt.

11. Vorrichtung nach Anspruch 7, wenn abhängig von Anspruch 4, **dadurch gekennzeichnet, dass** die Unterteilungs- und Modulationsgesetze, die in den Speichermitteln gespeichert sind, derart sind, dass sie ermöglichen, auf die einzelnen Impulsdauern der zweiten Phase in der Weise einzuwirken, dass die gesamte elektrische Ladung oder die gesamte Energie dieser zweiten Phase so dosiert wird, dass die elektrische Ladung bzw. die Energie der zweiten Phase einen gewünschten Anteil in Bezug auf die elektrische Ladung oder die Energie der ersten Phase hat, ohne die Dauer dieser zweiten Phase zu modifizieren.

12. Vorrichtung nach Anspruch 7, wenn abhängig von Anspruch 4, **dadurch gekennzeichnet, dass** die Unterteilungs- und Modulationsgesetze, die in den Speichermitteln gespeichert sind, derart sind, dass sie ermöglichen, auf die Dauern einzelner Pausen der zweiten Phase in der Weise einzuwirken, dass die gesamte elektrische Ladung oder die gesamte Energie dieser zweiten Phase dosiert wird, damit die elektrische Ladung bzw. die Energie der zweiten Phase einen gewünschten Anteil in Bezug auf die elektrische Ladung oder die Energie der ersten Phase hat, ohne die Dauer dieser zweiten Phase zu modifizieren.

13. Vorrichtung nach einem der Ansprüche 7, 9, 11 oder 12, **dadurch gekennzeichnet, dass** die Unterteilungs- und Modulationsgesetze, die in den Speichermitteln gespeichert sind, derart sind, dass die Dauer dieser zweiten Phase in der Weise gewählt werden kann, dass ihr Ladungs- oder Energiegehalt einen vorgegebenen Anteil in Bezug auf den Ladungs- bzw. Energiegehalt der ersten Phase hat.

14. Vorrichtung nach einem der vorhergehenden Ansprüche, **dadurch gekennzeichnet, dass** die Mittel zum Wählen eines Unterteilungsgesetzes dazu ausgelegt sind, dieses Gesetz im Verlauf des ersten Impulses zu wählen, und so beschaffen sind, dass der Takt dieses ersten Impulses ein bestimmtes Tastverhältnis in Bezug auf wenigstens ein anderes Tastverhältnis der Reihe von Impulsen besitzt.

15. Vorrichtung nach einem der Ansprüche 7 oder 9, **dadurch gekennzeichnet, dass** die Unterteilungs- und Modulationsgesetze, die in den Speichermitteln gespeichert sind, derart sind, dass die zweite Phase nicht in elementare Impulse unterteilt wird.

16. Vorrichtung nach Anspruch 15, **dadurch gekennzeichnet, dass** die Unterteilungs- und Modulationsgesetze, die in den Speichermitteln gespeichert sind, derart sind, dass die zweite nicht unterteilte Phase eine Dauer besitzt, derart, dass ihr Ladungs- oder Energiegehalt einen vorgegebenen Anteil in Bezug auf den Ladungs- bzw. Energiegehalt der ersten unterteilten Phase hat.

17. Vorrichtung nach einem der Ansprüche 14 bis 16, **dadurch gekennzeichnet, dass** die Unterteilungs- und Modulationsgesetze, die in den Speichermitteln gespeichert sind, derart sind, dass sie ermöglichen, auf das Tastverhältnis des Takts, der den ersten Impuls enthält, als Funktion des gemessenen Widerstands in der Weise einzuwirken, dass er einen vorgegebenen Wert hat.

18. Vorrichtung nach Anspruch 17, **dadurch gekennzeichnet, dass** die Unterteilungs- und Modulationsgesetze, die in den Speichermitteln gespeichert sind, derart sind, dass das Tastverhältnis des Takts, der den ersten Impuls enthält, mit dem Tastverhältnis wenigstens eines der folgenden Takte übereinstimmt.
